# EUROPEAN PATENT SPECIFICATION

(11) **EP 3 364 866 B1**
(45) Date of publication and mention of the grant of the patent: **24.02.2021**
(21) Application number: 15891401.0
(22) Date of filing: 23.10.2015
(51) Int. Cl.: A61B 5/026, A61B 5/021, A61B 5/00, A61B 5/0215, A61B 5/15, A61M 25/02, A61M 39/02, A61M 25/00

(54) **INTEGRATED PRESSURE SENSING DEVICE**
INTEGRIERTE DRUCKMESSUNGSVORRICHTUNG
DISPOSITIF CAPTEUR DE PRESSION INTÉGRÉ

(43) Date of publication of application: 29.08.2018
(73) Proprietor: Edwards Lifesciences Corporation, Irvine, CA 92614 (US)
(72) Inventor: SIEMONS, Alexander, H., Irvine, CA 92614 (US); BACKER, Steven, E., Irvine, CA 92614 (US)
(74) Representative: Eisenführ Speiser
(86) International application number: PCT/US2015/057225
(87) International publication number: WO 2017/069783

(56) References cited:
- US-A- 5 037 398
- US-A- 5 769 083
- US-A- 6 139 503
- US-A1- 2003 208 127
- US-A1- 2004 143 262
- US-A1- 2006 144 155
- US-A1- 2010 056 932
- US-A1- 2010 298 777

## Description

### BACKGROUND

When diagnosing and treating various bodily ailments, such as with patients suffering from shock or cardiovascular problems, medical personnel often find it desirable to measure or monitor a patient's blood pressure. By measuring and monitoring the blood pressure of these and other types of patients, medical personnel are better able to detect blood flow difficulties and other cardiovascular problems at an early stage. As a result, the use of blood pressure measurement and monitoring may increase the likelihood that a patient can be successfully treated or provided with needed emergency assistance.

A variety of methods is currently used for measuring and monitoring blood pressure. For example, medical personnel frequently use various indirect blood pressure measurement techniques, such as measuring a patient's blood pressure by using a pressure cuff and a stethoscope. In addition, blood pressure measurements are often made using a number of direct measurement and monitoring techniques. Notably, when diagnosing or treating critically ill patients, such direct techniques are often preferred over any of the indirect techniques. Direct blood pressure measurement and monitoring techniques are generally more accurate and facilitate the continuous monitoring of a patient's blood pressure on a beat-to-beat basis. Direct blood pressure monitoring also enables the rapid detection of a change in cardiovascular activity, and this may be of significant importance in emergency situations.

In at least some direct, invasive, blood pressure monitoring systems, a catheter is inserted into a patient's circulatory system with the end of the catheter having an opening to the blood stream, typically in a major or peripheral blood vessel. An IV set attaches to the proximal end of the catheter protruding from the patient so that a solution flows through the catheter and into the patient. The IV solution provides a fluid column through which pressure pulses are transmitted, and a pressure transducer positioned along the fluid column monitors those pressure pulses. Disposable blood pressure transducers (DPTs) that connect to a monitor are often used in the OR, ICU or CCU of a medical care facility. Due to the separable nature of the transducer and monitor, different transducers may be connected to any one monitor, as long as cable connectors' electrical interfaces are compatible.

US patent 5,037,398 relates to a fastening system for securing a pressure monitor device to a patient. The system comprises an adhesive sheet constructed and arranged to the base of the device and carry the device, and at least one strap which folds over and releasably secures the device to the sheet, and a releasable fastener which releasably secures the base of the device.

US patent 6,139,503 describes a pressure transducer arrangement featuring a holding element for a number of pressure transducers. For each pressure transducer a group of electronic counter-contacts is provided on the holding element that are in contact with the contacts of the pressure transducer while it is attached to the holding element. All counter-contacts are connected to one common signal transfer device.

US patent 5,769,083 relates to an optical system for positioning a pressure transducer relative to a patient to provide for accurate measurement of bodily fluids within the patient. A light source such as a laser is used to direct light onto a specified location on the human body to accurately identify the elevation of the transducer relative to the specified location to provide an absolute measurement of fluid pressure.

### SUMMARY

Embodiments of the disclosed concepts provide a device that integrates multiple hemodynamic pressure transducers or sensors into a single device package. The sensor package can include a mounting substrate designed to be attached to a monitored subject and placed approximately near the heart of the subject. Stated differently, the device can be designed for precordial mounting to the subject so that pressure readings are substantially accurate and appropriately calibrated without "leveling" the sensors relative to a subject's heart using a separate support, and regardless of the subject's position. In some embodiments, the assembly, including all transducers, can be fed through a single lumen such as a tube to an IV bag.

A hemodynamic sensing device according to at least some embodiments includes a precordial mounting substrate and a plurality of pressure transducers configured to be attached to the precordial mounting substrate while the precordial mounting substrate is secured to a subject such as a hospital patient. An electrical interface is provided for the plurality of pressure transducers. The electrical interface is adapted to communicate an electrical signal from the plurality of pressure transducers to a receiving device such as a bedside medical monitor. A lumen set is configured to provide fluid to the plurality of pressure transducers from subject's blood stream.

In some embodiments the pressure transducers or sensors are adapted to be selectively attached to a mounting substrate. Alternatively, sensing devices having various numbers of sensors can be manufactured and selected as appropriate by medical personnel. In some embodiments, the hemodynamic sensing device includes a single feed lumen to provide fluid for the fluid columns for all of the plurality of pressure transducers, thus reducing the number of supply tubes necessary to use the device.

In some embodiments, the device includes a blood draw port connected to the lumen set so that routine blood testing can be carried out without creating an additional site on the subject being monitored. In at least some embodiments, the mounting substrate for the pressure transducers is or includes an adhesive pad for positioning on the subject's skin at or near the level of the heart. The integrated sensor device package can also include a fastening mechanism to mount the device to a subject's clothes instead of directly to the subject. In example embodiments, the electrical signaling to a receiving system or device can be provided through a cable either with or without a detachable connector, or through a wireless transmitter.

As previously mentioned, a device according to example embodiments can be provided in a modular form so that the requisite number of transducers can be assembled together in the field to meet individual need. Alternatively, sensor device packages can be pre-assembled. In either case, to use the device, a pressure sensor or pressure sensors can be attached to the mounting substrate, which is designed to be attached to a subject at approximately heart level. A sensor can be connected to an electrical interface to be operable to communicate an electrical signal to a receiving device, and a lumen set can be connected to be operable to maintain a fluid column between a pressure sensor and the subject. When used with an appropriate monitor, these components provide the means for medical personnel to monitor a patient's pressure at various points.

In some embodiments of the concepts disclosed herein, a single support substrate connects a plurality of pressure transducers together and provides for those transducers to be connected to an electrical interface to communicate electrical signals from the transducers to a receiving device. A lumen set is configurable to provide fluid to the plurality of pressure transducers from the subject's blood stream. In this manner, an integrated device can be provided so that medical personnel do not need to deal routinely with as many individual sensor devices and connections as might otherwise be the case. In some embodiments, the single support substrate is configured to be precordially mounted. The integrated hemodynamic sensing device can include a plurality of pressure transducers that are adapted to be selectively attachable to the single support substrate. The invention is defined by appended claims 1-10.

### BRIEF DESCRIPTION OF THE DRAWINGS

FIG. 1 is a relatively close-up perspective view of a device according to example embodiments of the concepts disclosed.
FIG. 2 is a perspective view of a device according to example embodiments. In the view shown in FIG. 2, the tubing and an electrical connecting cable are visible.
FIG. 3 illustrates a device according to additional embodiments wherein the sensor package includes a blood draw port.
FIG. 4 illustrates a device according to example embodiments as it would be placed on a subject being monitored.
FIG. 5 is a schematic, perspective view of a device according to an embodiment in which the device is provided in modular or kit form.

### DETAILED DESCRIPTION OF EXAMPLE EMBODIMENTS

The term "sensor" as used herein relates to a device, component, or region of a device capable of detecting, quantifying, or qualifying a sensed physical property in the body of a subject. A disposable pressure transducer (DPT) is a sensor that typically includes an identifier that can be detected by an associated monitor such that the monitor recognizes the characteristics of the device. If the monitor recognizes the type of DPT as compatible, it can proceed with the pressure measurement. The term "substrate" is meant in a broad sense in that the term is intended to not only encompass a flat article onto which sensors or sensor housings can be fixed, but also a housing which may cover all the sensors or sensor assemblies to integrate them into a single device package and provide a means for operatively connecting a plurality of pressure transducers together to form an integrated device.

A pressure transducer is a sensor capable of sensing or determining a pressure such as the fluid pressure within a lumen leading out of the body from an arterial catheter, and converting such pressure to a signal that can be communicated to a receiving device such as a monitor or display. A number of such pressure transducers are known. None are specifically required and many could be used in an embodiment of the concepts described herein. Blood pressure can be sensed directly (i.e., via direct physical contact with blood or IV fluid) or indirectly (i.e., optically or at a pulse point through the skin). For example, catheter-based fluid pressure transducers in direct contact with arterial blood or a fluid column in contact with arterial blood are presently accepted as standard practice in the OR, ICU or CCU, though less-invasive techniques such as external piezo-electric sensors in contact with the skin are available.

With some embodiments of the disclosed concepts, an IV set attaches to the proximal end of the catheter protruding from the patient so that a solution flows through the catheter and into the patient. The IV solution provides the fluid column through which pressure pulses are transmitted, and a pressure transducer positioned along the fluid column senses the changing pressure to provide hemodynamic monitoring. Generally, the pressure transducer can consist of a dome that functions as a reservoir for the IV fluid. The dome includes a resilient diaphragm that attaches to an electrical transducer component. The transducer senses pressure fluctuations in the diaphragm and converts them into electrical signals, which are then transmitted through a cable to a receiving device such as a monitor for amplification and display. A single silicon chip inside the transducer can include both the pressure diaphragm and the measuring circuitry of the pressure transducer. Since such silicon chips can be cheaply mass-produced, the total cost of pressure transducers is such that the transducer may be economically disposable. The cable includes a connector so that the transducer and associated portion of the cable can be discarded after use, whereas the mating connector and cable hard-wired to the monitor can be reused. Such disposable blood pressure transducers (DPTs) are the standard of care in the OR, ICU or CCU. Compatibility of a DPT with a given receiving system is assured through the use of standards. For example, the International Electrotechnical Commission includes a standard for the DPT interface, IEC 60601-2-34.

In order to maintain accuracy in pressure readings from pressure transducers that sense pressure in a fluid column as described above, siphonic pressure differences between the patients circulatory system and the sensor must be minimized. This can be practically accomplished by "zeroing" or "leveling" the pressure transducer so that it is physically at the same or at least substantially the same level as the subject's heart.

There is often a need to monitor pressure at various locations in the body. Thus multiple fluid columns are provided via a lumen set, which may be implemented by a plurality of plastic IV tubes. The sensors with the IV tubes attached can be mounted on a movable bracket that is manually adjusted when the patient moves or is moved by medical personnel. Alternatively, embodiments of the concepts disclosed below can provide a system where the transducers self level by automatically moving with the monitored subject. A device that includes the sensors and the mechanism for leveling the sensors may be called herein a "pressure sensing" device, a "blood pressure sensing" device, a "hemodynamic monitoring" device, a "pressure monitoring" device, or any other similar term may be used.

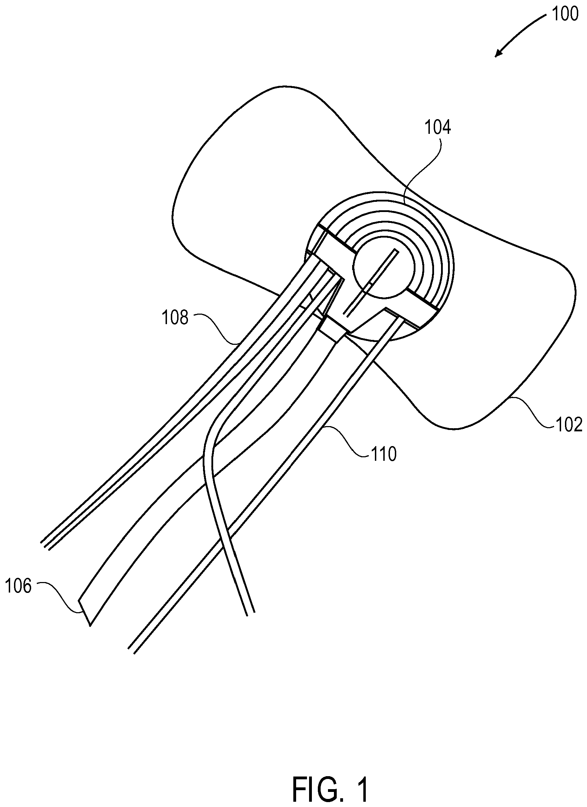

FIG. 1 is a relatively close-up perspective view of a self-leveling, integrated hemodynamic sensing device according to example embodiments of the concepts disclosed herein. Device 100 includes a precordial mounting substrate 102. A plurality of pressure transducer assemblies 104 are attached to the precordial mounting substrate. An electrical interface, including ribbon cable 106 carries wires to the pressure transducer assemblies 104. A pressure transducer assembly includes a positioning housing, which can accept fluid and includes the transducer component itself mounted inside the positioning housing so that the positioning housing provides at least a portion of the means to provide fluid to the plurality of transducers. The wires leading from the transducer assemblies ultimately connect back to a receiving device such as a bedside monitor. Lumen set 108, implemented via a plurality of IV tubes, maintains a fluid column between each pressure transducer and the subject's bloodstream. In this particular case, the lumen set includes three tubes, and the device includes three transducer assemblies 104 ganged together and attached to precordial mounting substrate 102. Thus, multiple, independent pressure transducers can be replaced with a single integrated package.

Still referring to FIG. 1, device 100 also includes a single feed lumen to provide fluid for the fluid columns for all of the pressure transducers in the device. The single feed lumen is implemented by IV tube 110. In this example embodiment, the precordial mounting substrate 102 is includes an adhesive pad. This adhesive pad can be placed on the subject at heart level, so that pressure transducer assemblies 104 move with the subject and remain "zeroed" relative to the subject's heart, eliminating errors caused by siphonic pressure differences. It should be noted that the electrical interface can be provided wirelessly as well as through a cable. Any of various standards could be used for wireless connectivity, for example, ANT, Bluetooth, or Wi-Fi. With some wireless connections, the pressure sensing device and lumens could be located remotely from a connected display monitor, even at a significant distance. In FIG. 1, the pressure transducers themselves are not visible inside the transducer assemblies; however they could be if the housings of the assemblies were made a transparent material, as shown in additional embodiments discussed below.

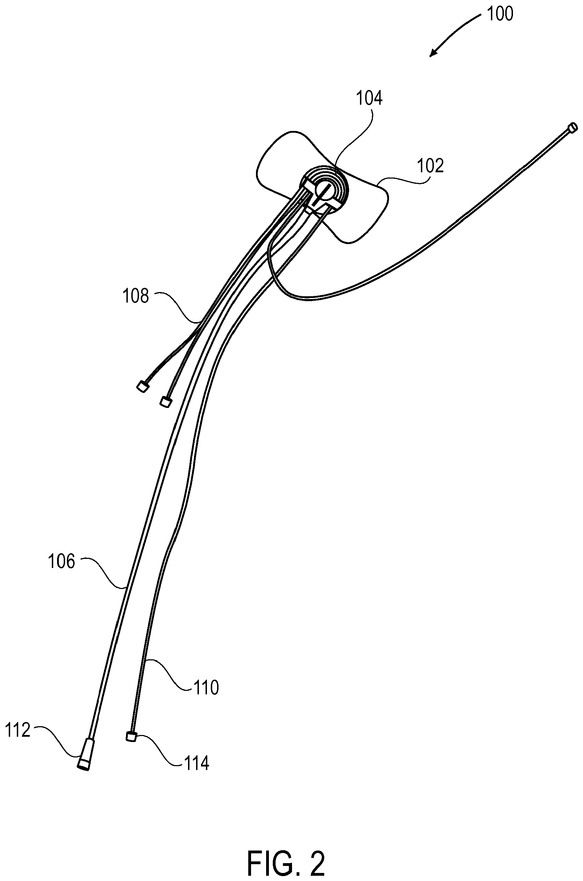

FIG. 2 is another view of hemodynamic sensing device 100. In FIG. 2, a perspective from further away from the device is presented to the reader. Like reference numbers are used, since many of the components that are visible are the same components that were visible in FIG. 1. However, cable 106 can be seen all the way to its end, where electrical connector 112 also forms a part of the electrical interface for the pressure transducer assemblies 104. Also visible in FIG. 2 is a connector 114 for an IV bag to provide a fluid reservoir for feed lumen 110.

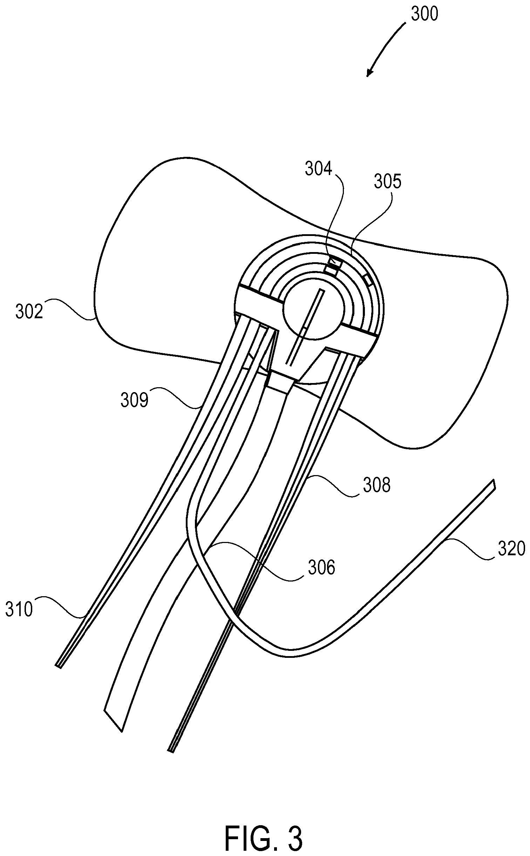

FIG. 3 illustrates another device according to embodiments of the concepts disclosed herein. Device 300 includes a precordial mounting substrate 302. A plurality of pressure transducers 304 are disposed inside pressure transducer assemblies 305, which are attached to the precordial mounting substrate and include transparent positioning housings. Ribbon cable 306 carries wires to the pressure transducers. The lumen set that maintains a fluid column between each pressure transducer and the subject's bloodstream in this particular case includes four tubes, and the device includes four transducer assemblies 305 ganged together and attached to precordial mounting substrate 102. Tubes 308 implement three lumens. In this case, the outer tube 309 implements the other lumen from the lumen set to provide fluid to the fourth transducer.

Still referring to FIG. 3, pressure or hemodynamic sensing device 300 also includes a single feed lumen to provide fluid for the fluid columns for all of the pressure transducers in the device. The single feed lumen is implemented by IV tube 310, which in this case protrudes from the device on the same side as a tube that provides fluid to one of the four transducers. In this example embodiment, the precordial mounting substrate 302 is or includes an adhesive pad that can be placed on the subject at heart level to remain "zeroed" relative to the subject's heart. Device 300 of FIG. 3 also includes an optional blood draw port 320 internally connected to the lumen set. This blood draw port allows routine blood testing on the subject being monitored to be carried out without creating an additional access site. Thus, again, multiple, independent pressure transducers can be replaced with a single integrated package. Further, in the embodiment of FIG. 3, a blood draw port can be included in the integrated package.

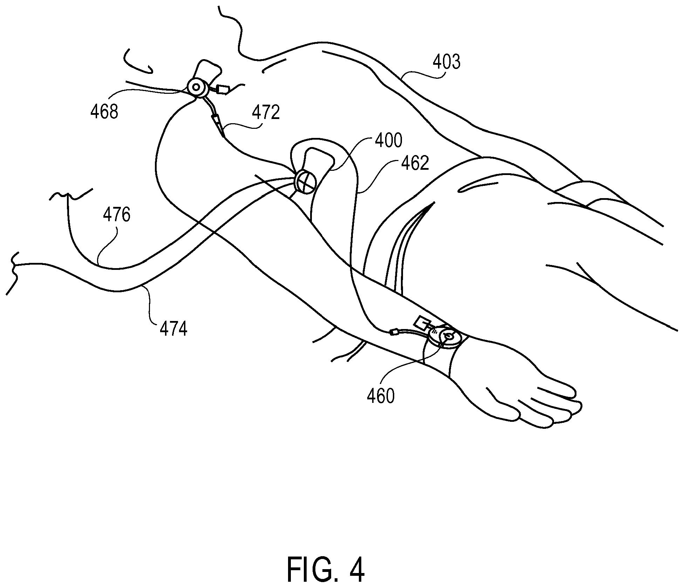

FIG. 4 is a schematic illustration of a device according to example embodiments, as it would be placed on a subject being monitored. In FIG. 4, self-leveling pressure sensing device 400 like those described above is placed on subject 403 in the precordial area to be substantially at the same level as the heart. A pressure transducer in device 400 monitors pressure in a radial artery in the patient's right arm at site 460. Lumen 462 provides a fluid column between site 460 and the transducer in pressure sensing device 400. Similarly, another pressure transducer in device 400 monitors pressure in a carotid artery in the patient's neck at site 468. Lumen 472 provides a fluid column between site 460 and the transducer in pressure sensing device 400. In the example of FIG. 4, the electrical interface to a bedside monitor (not shown) is via electrical cable 474 and feed lumen 476 provides fluid for the fluid columns in lumens 462 and 472 to allow the pressure transducers to send pressure in subject 403. Device 400 is an integrated device with a plurality of transducers packaged together, reducing the number of wires and tubes, making for a cleaner appearance and thus improving patient comfort.

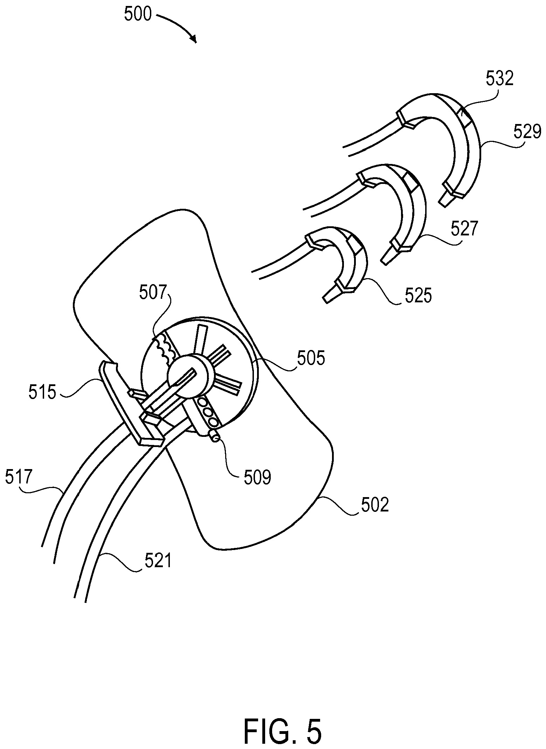

FIG. 5 illustrates a hemodynamic monitoring device provided in modular, kit form so that the requisite number of pressure transducers can be assembled together in the field to meet individual need. Device 500 of FIG. 5 includes precordial mounting substrate 502. Substrate 502 may again be or include an adhesive pad, or other fasteners that could be used as a means for attaching the device on the subject or the subject's clothing at approximately heart level. Precordial mounting substrate 502 includes a mounting bracket 505 and lumen management brackets 507 and 509 to at least in part provide a means to selectively connect at least some of the plurality of pressure transducers to form an integrated device in the field. Strain relief 515 supports electrical cable 517. A single feed lumen is implemented by IV tube 521. Disposable pressure transducer assemblies 525, 527, and 529 can be snapped into bracket 505 to create a one, two, or three transducer system. Each transducer assembly includes a physical pressure transducer device 532. Due to the radial design of device 500, transducer 525 may necessarily be the first transducer, transducer 527 may necessarily be the second transducer, and transducer 529 may necessarily be the third transducer. A fourth transducer could be installed in some kits, as could a blood draw port. Device 500 of FIG. 5 is but an example only. Modular hemodynamic monitoring devices can be engineered so that all the transducer assemblies are the same size and are interchangeable.

## Claims

1. A hemodynamic sensing device (100) comprising:
a precordial mounting substrate (102);
a plurality of pressure transducers (104) configured to be attached to the precordial mounting substrate (102) while the precordial mounting substrate (102) is secured to a subject;
an electrical interface (106) for the plurality of pressure transducers, the electrical interface adapted to communicate an electrical signal from the plurality of pressure transducers (104) to a receiving device; and
a lumen set (108) configured to provide fluid to the plurality of pressure transducers (104) from the subject's blood stream.

2. The hemodynamic sensing device (100) of claim 1 wherein the plurality of pressure transducers (104) are adapted to be selectively attached to the precordial mounting substrate (102).

3. The hemodynamic sensing device (100) of claim 1 further comprising a single feed lumen to provide fluid for all of the plurality of pressure transducers (104).

4. The hemodynamic sensing device (100) of claim 1 further comprising a blood draw port connected to the lumen set (108).

5. The hemodynamic sensing device (100) of claim 1 wherein the precordial mounting substrate (102) further comprises an adhesive pad.

6. The hemodynamic sensing device (100) of claim 1 wherein the electrical interface (106) further comprises at least one of an electrical connector, an electrical cable, and a wireless transmitter.

7. A method of providing a hemodynamic sensing device, the method comprising:
configuring a mounting substrate (102) to be attached to a subject at approximately heart level;
attaching a plurality of pressure transducers (104) to the mounting substrate (102) to form an integrated device package;
connecting the plurality of pressure transducers (104) to an electrical interface (106) to be operable to communicate an electrical signal from the plurality of pressure transducers (104) to a receiving device; and
connecting a lumen set (108) to the plurality of pressure transducers (104) to be operable to maintain a fluid column between each of the plurality of pressure transducers (104) and the subject's blood stream.

8. The method of claim 7 further comprising connecting a single feed lumen to the plurality of pressure sensors.

9. The method of claim 7 wherein the mounting substrate (102) is an adhesive pad.

10. The method of claim 9 wherein the electrical interface (106) comprises at least one of an electrical connector, an electrical cable, and a wireless transmitter.

## Patentansprüche

1. Hämodynamische Messungsvorrichtung (100), umfassend:
ein präkordiales Montagesubstrat (102);
eine Vielzahl von Druckaufnehmern (104), die zur Anbringung an dem präkordialen Montagesubstrat (102) konfiguriert ist, während das präkordiale Montagesubstrat (102) an einem Subjekt gesichert ist;
eine elektrische Schnittstelle (106) für die Vielzahl von Druckaufnehmern, wobei die elektrische Schnittstelle dazu ausgelegt ist, ein elektrisches Signal von der Vielzahl von Druckaufnehmern (104) an eine Empfangsvorrichtung zu kommunizieren; und
einen Lumensatz (108), der dazu konfiguriert ist, der Vielzahl von Druckaufnehmern (104) ein Fluid aus dem Blutkreislauf des Subjekts bereitzustellen.

2. Hämodynamische Messungsvorrichtung (100) nach Anspruch 1, wobei die Vielzahl von Druckaufnehmern (104) dazu ausgelegt ist, selektiv an dem präkordialen Montagesubstrat (102) angebracht zu werden.

3. Hämodynamische Messungsvorrichtung (100) nach Anspruch 1, ferner umfassend ein Lumen mit einer einzigen Zuleitung, um Fluid für alle der Vielzahl von Druckaufnehmern (104) bereitzustellen.

4. Hämodynamische Messungsvorrichtung (100) nach Anspruch 1, ferner umfassend einen Blutentnahmeanschluss, der mit dem Lumensatz (108) verbunden ist.

5. Hämodynamische Messungsvorrichtung (100) nach Anspruch 1, wobei das präkordiale Montagesubstrat (102) ferner ein Klebepad umfasst.

6. Hämodynamische Messungsvorrichtung (100) nach Anspruch 1, wobei die elektrische Schnittstelle (106) ferner mindestens eines von einem elektrischen Verbinder, einem elektrischen Kabel und einem drahtlosen Sender umfasst.

7. Verfahren zum Bereitstellen einer hämodynamischen Messungsvorrichtung, wobei das Verfahren Folgendes umfasst:
Konfigurieren eines Montagesubstrats (102) zum Anbringen an einem Subjekt auf etwa der Höhe des Herzens;
Anbringen einer Vielzahl von Druckaufnehmern (104) an dem Montagesubstrat (102), um ein integriertes Vorrichtungspaket zu bilden;
Verbinden der Vielzahl von Druckaufnehmern (104) mit einer elektrischen Schnittstelle (106), um sie zum Kommunizieren eines elektrischen Signals von der Vielzahl von Druckaufnehmern (104) an eine Empfangsvorrichtung betreiben zu können; und
Verbinden eines Lumensatzes (108) mit der Vielzahl von Druckaufnehmern (104), um ihn zum Aufrechterhalten einer Fluidsäule zwischen jedem der Vielzahl von Druckaufnehmern (104) und dem Blutkreislauf des Subjekts betreiben zu können.

8. Verfahren nach Anspruch 7, ferner umfassend das Verbinden eines Lumens mit einer einzigen Zuleitung mit der Vielzahl von Drucksensoren.

9. Verfahren nach Anspruch 7, wobei es sich bei dem Montagesubstrat (102) um ein Klebepad handelt.

10. Verfahren nach Anspruch 9, wobei die elektrische Schnittstelle (106) mindestens eines von einem elektrischen Verbinder, einem elektrischen Kabel und einem drahtlosen Sender umfasst.

## Revendications

1. Dispositif de détection hémodynamique (100) comprenant :
un substrat de montage précordial (102) ;
une multitude de transducteurs de pression (104) configurés pour être attachés au substrat de montage précordial (102) tandis que le substrat de montage précordial (102) est fixé à un patient ;
une interface électrique (106) pour la multitude de transducteurs de pression, l'interface électrique conçue pour communiquer un signal électrique de la multitude de transducteurs de pression (104) à un dispositif récepteur ; et
un ensemble de lumen (108) configuré pour fournir un fluide à la multitude de transducteurs de pression (104) du flux sanguin du patient.

2. Dispositif de détection hémodynamique (100) selon la revendication 1, dans lequel la multitude de transducteurs de pression (104) sont conçus pour être attachés sélectivement au substrat de montage précordial (102).

3. Dispositif de détection hémodynamique (100) selon la revendication 1 comprenant en outre un lumen à alimentation unique afin de fournir du fluide pour chacun de la multitude de transducteurs de pression (104).

4. Dispositif de détection hémodynamique (100) selon la revendication 1 comprenant en outre un orifice de prise de sang relié à l'ensemble de lumen (108).

5. Dispositif de détection hémodynamique (100) selon la revendication 1, dans lequel le substrat de montage précordial (102) comprend en outre une pastille adhésive.

6. Dispositif de détection hémodynamique (100) selon la revendication 1, dans lequel l'interface électrique (106) comprend en outre au moins un d'un connecteur électrique, d'un câble électrique et d'un transmetteur sans fil.

7. Procédé permettant de fournir un dispositif de détection hémodynamique, le procédé comprenant :
la configuration d'un substrat de montage (102) pour être attaché à un patient approximativement au niveau du coeur ;
l'attache d'une multitude de transducteurs de pression (104) au substrat de montage (102) afin de former un boîtier de dispositif intégré ;
le lien de la multitude de transducteurs de pression (104) à une interface électrique (106) pour pouvoir communiquer un signal électrique de la multitude de transducteurs de pression (104) à un dispositif récepteur ; et
le lien d'un ensemble de lumen (108) à la multitude de transducteurs de pression (104) afin de pouvoir maintenir une colonne de fluide entre chacun de la multitude de transducteurs de pression (104) et le flux sanguin du patient.

8. Procédé selon la revendication 7 comprenant en outre un lien d'un lumen à alimentation unique à la multitude des capteurs de pression.

9. Procédé selon la revendication 7, dans lequel le substrat de montage (102) est une pastille adhésive.

10. Procédé selon la revendication 9, dans lequel l'interface électrique (106) comprend au moins un d'un connecteur électrique, d'un câble électrique et d'un transmetteur sans fil.
